# EUROPEAN PATENT APPLICATION

(11) **EP 4 721 722 A1**
(43) Date of publication of application: **08.04.2026**
(21) Application number: 25823736.1
(22) Date of filing: 17.06.2025
(51) Int. Cl.: A61K 8/06, A61K 8/39, A61K 8/37, A61K 8/26, A61K 8/49, A61K 8/41, A61K 8/46, A61K 8/895

(54) **COSMETIC COMPOSITION EXHIBITING EXCELLENT BLOCKING EFFICACY AGAINST HARMFUL RAYS INCLUDING ULTRAVIOLET RADIATION**

(30) Priority: 19.06.2024 KR 20240079949
(71) Applicant: Kolmar Korea Co., Ltd., Sejong 30004 (KR)
(72) Inventor: KIM, Jin Young, Seoul 06800 (KR); SOHN, Ji Hyun, Seoul 06800 (KR); LEE, Ye Rin, Seoul 06800 (KR)
(74) Representative: Nederlandsch Octrooibureau
(86) International application number: PCT/KR2025/008365
(87) International publication number: WO 2025/263966

(57) **Abstract**

A cosmetic composition of the present invention, which has an excellent effect of blocking harmful radiation including ultraviolet radiation, contains at least three UV blockers, an emulsifier, and a thickener, and thus may exhibit an excellent effect of blocking harmful radiation including ultraviolet radiation by stabilizing the at least three UV blockers in the formulation. In addition, the cosmetic composition has excellent long-lasting water resistance, and thus it may continuously exhibit an excellent effect of blocking harmful radiation even when the user perspires or engages in activities in water.

## Description

### Technical Field

The present invention relates to a cosmetic composition having an excellent effect of blocking harmful radiation including ultraviolet (UV) radiation, and more specifically, to a cosmetic composition in emulsion form having an excellent effect of blocking harmful radiation by stably containing at least three UV blockers in the formulation.

### Background Art

Electromagnetic waves may be classified according to their wavelengths into infrared radiation, visible light, and ultraviolet (UV) radiation. Among them, UV radiation to which humans are exposed through sunlight has short wavelengths and strong energy, and thus even short-term exposure thereto can cause problems such as skin aging and wrinkles. Among UV radiation, ultraviolet A (UVA; UV radiation with a wavelength of 320 to 400 nm), which is classified as a long-wavelength UV radiation, is known to reach the dermis layer of the skin due to its longer wavelength than other UV radiation, tanning the skin and causing long-term skin damage due to aging, and ultraviolet B (UVB; UV radiation with a wavelength of 280 to 320 nm), a radiation that has direct adverse effects on the skin, tans the skin and causes skin cancer. Ultraviolet C (UVC; UV radiation with a wavelength of less than 280 nm), a UV radiation with the shortest wavelength, has the highest energy, and the amount that reaches the Earth's surface is very small because UVC is completely absorbed by the ozone layer, but it is known that UVC causes burns, skin cancer, or cataracts when the human body is exposed thereto. Blue light, a type of visible light, has a wavelength of about 400 to 500 nm, has the strongest energy among light that reaches the retina, and may have a harmful effect on the skin when exposed thereto for a long period of time.

Since there are various types of harmful radiation that are harmful to the skin as described above, protective measures against a wide range of wavelengths are required.

Commercially widely available water-in-oil type UV blocking products either contain only inorganic UV blockers or contain a mixture of organic and inorganic UV blockers. Organic UV blockers are generally oil-soluble, and thus applying UV blockers to the aqueous phase part is uncommon. In addition, thickeners are contained to ensure an appropriate formulation, and in this case, they are mostly oil-soluble thickeners applied to the oil phase part.

The present inventors have conducted extensive studies to effectively block a wide range of electromagnetic waves through a single product, and as a result, have developed a cosmetic composition capable of effectively blocking a wide range of harmful radiation by stably containing an organic or inorganic UV blocker in both the aqueous phase part and the oil phase part.

### DISCLOSURE

### Technical Problem

An object of the present invention is to provide a cosmetic composition having an excellent effect of blocking harmful radiation including UV radiation.

The technical problems of the present invention are not limited to the technical problems mentioned above, and other technical problems not mentioned may be clearly understood by those skilled in the art from the following description.

### Technical Solution

According to an embodiment of the present invention, there is provided a cosmetic composition in emulsion form including an aqueous phase part and an oil phase part, the cosmetic composition containing at least three UV blockers, an emulsifier, and a thickener, wherein the at least three UV blockers include a first UV blocker, a second UV blocker, and a third UV blocker, thereby providing an excellent effect of blocking harmful radiation including UV radiation.

### Advantageous Effects

The cosmetic composition of the present invention stably contains at least three UV blockers in the formulation, and thus may exhibit not only an excellent blocking effect against UV radiation (UVA, UVB, and UVC) in a wide range of wavelengths, but an excellent effect of blocking other harmful radiation (such as blue light) including UV radiation.

In addition, the cosmetic composition contains a UV blocker component in both the aqueous phase part and the oil phase part, and thus may reduce the variation in the harmful radiation blocking effect depending on the size of emulsion particles, thereby exhibiting a uniform effect of blocking harmful radiation.

In addition, the cosmetic composition has excellent long-term water resistance, and thus may exhibit an excellent effect of blocking harmful radiation for a long period of time from the time of application to the skin.

### Brief Description of Drawings

In order to more fully understand the drawings referred to in the detailed description of the present invention, a brief description of each drawing is provided.

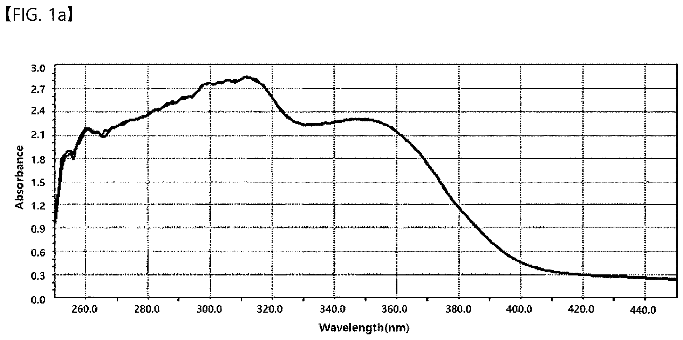

FIG. 1a shows the absorbance of Example 1 for harmful radiation with a wavelength ranging from 250 to 450 nm.

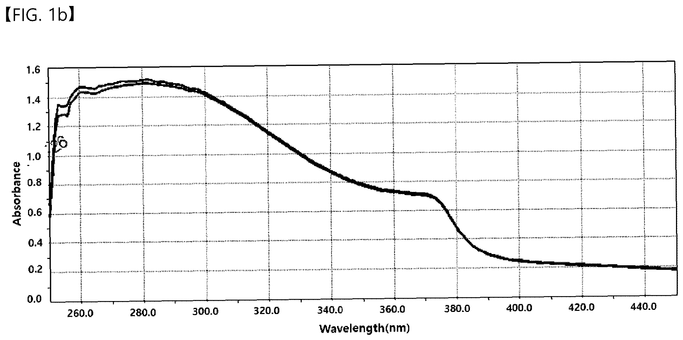

FIG. 1b shows the absorbance of Comparative Example 1 for harmful radiation with a wavelength ranging from 250 to 450 nm.

### Best Mode

According to an embodiment of the present invention, there is provided a cosmetic composition in emulsion form including an aqueous phase part and an oil phase part, the cosmetic composition containing at least three UV blockers, an emulsifier, and a thickener, wherein the at least three UV blockers include a first UV blocker, a second UV blocker, and a third UV blocker, thereby providing an excellent effect of blocking harmful radiation including UV radiation.

In addition, the first UV blocker may be contained in the aqueous phase part.

In addition, the second UV blocker and the third UV blocker may be contained in the oil phase part.

In addition, the emulsifier and the thickener may be present in a weight ratio from 0.5:1 to 10:1.

In addition, the emulsifier may include a polyglyceryl-based emulsifier.

In addition, the polyglyceryl-based emulsifier may include at least one of polyglyceryl-5 polyricinoleate, polyglyceryl-6 polyricinoleate, polyglyceryl-2 dipolyhydroxystearate, polyglyceryl-4 isostearate, polyglyceryl-4 diisostearate/polyhydroxystearate/sebacate, polyglyceryl-2 oleate, polyglyceryl-4 oleate, polyglyceryl-6 oleate, polyglyceryl-2 stearate, and polyglyceryl-4 stearate.

In addition, the emulsifier may be contained in an amount of 0.1 to 7.0 wt% based on the total weight of the cosmetic composition.

In addition, the thickener may be contained in the aqueous phase part.

In addition, the thickener may include at least one of saponite, montmorillonite, smectite, kaolin, illite, hectorite, bentonite, and magnesium aluminum silicate.

In addition, the thickener may be contained in an amount of 0.01 to 3.0 wt% based on the total weight of the cosmetic composition.

In addition, the first UV blocker may include at least one of methylene bis-benzotriazolyl tetramethylbutylphenol, tris-biphenyl triazine, phenylbenzimidazole sulfonic acid, terephthalylidene dicamphor sulfonic acid, titanium dioxide, and bis-ethylhexyloxyphenol methoxyphenyl triazine.

In addition, the second UV blocker may include at least one of ethylhexyl triazone, diethylamino hydroxybenzoyl hexyl benzoate, bis-ethylhexyloxyphenol methoxyphenyl triazine, ethylhexyl methoxycinnamate, ethylhexyl salicylate, octocrylene, diethylhexyl butamido triazone, homosalate, isoamyl p-methoxycinnamate, and polysilicone-15.

In addition, the third UV blocker may include at least one of titanium dioxide and zinc oxide.

In addition, the harmful radiation including UV radiation may have a wavelength of 250 to 500 nm.

### Mode for Invention

Unless otherwise defined, all technical and scientific terms used in the present specification have the same meanings as commonly understood by those skilled in the art to which the present disclosure pertains. In general, the nomenclature used in the present specification is well known and commonly used in the art. In addition, when describing embodiments of the present invention, if it is determined that specific description of a related known configuration or function hinders the understanding of the embodiments of the present invention, the detailed description thereof will be omitted. In addition, although embodiments of the present invention will be described below, the technical idea of the present invention is not limited or restricted thereto and may be modified and implemented in various ways by those skilled in the art.

In the present specification, it is understood that when any part is referred to as including any component, it does not exclude other components, but may further include other components, unless specifically stated otherwise. In the present specification, the term "and/or" includes a combination of a plurality of related items or any one of a plurality of related items.

According to an embodiment of the present invention, there is provided a cosmetic composition in emulsion form having an excellent effect of blocking harmful radiation including UV radiation. The cosmetic composition of the present invention contains at least three UV blockers, an emulsifier, and a thickener, wherein the at least three UV blockers include a first UV blocker, a second UV blocker, and a third UV blocker. Specifically, the cosmetic composition is characterized by having an excellent blocking effect against harmful radiation in a wide range of wavelengths by stabilizing the at least three UV blockers in the emulsion formulation through a combination of the emulsifier and the thickener.

In the present specification, harmful radiation including UV radiation refers to light radiation including all ultraviolet A (UVA; UV radiation with a wavelength of 320 to 400 nm), ultraviolet B (UVB; UV radiation with a wavelength of 280 to 320 nm), ultraviolet C (UVC; UV radiation with a wavelength of 250 to 280 nm) in a certain wavelength range, and blue light (blue visible light with a wavelength of 400 to 500 nm). The cosmetic composition of the present invention has a blocking effect against blue light with a wavelength of about 400 to 500 nm, and thus has the effect of blocking a wide range of harmful radiation in a wavelength ranging from 250 to 500 nm.

In one embodiment, the first UV blocker may be contained in the aqueous phase part. While a UV blocker component is generally applied to the oil phase part in a cosmetic composition in emulsion form, the cosmetic composition in emulsion form according to an embodiment of the present invention may have a uniform and excellent blocking effect against harmful radiation by containing the first UV blocker component stably applied to the aqueous phase part.

In a specific embodiment, the aqueous phase part of the cosmetic composition in emulsion form may contain a first UV blocker and a thickener, and the oil phase part may contain a second UV blocker and a third UV blocker. If the UV blocker component in a cosmetic composition in emulsion form is applied only to the oil phase part, there may be a local variation in the blocking effect against harmful radiation depending on the size of emulsion particles in the formulation. However, the cosmetic composition in emulsion form according to an embodiment of the present invention may have a uniform and excellent blocking effect against harmful radiation as it contains at least three UV blockers stably applied to the aqueous phase part and the oil phase part. In addition, as the thickener is applied to the aqueous phase part, the UV blocker may be stabilized within the formulation and may contribute to the formation and stabilization of emulsion particles.

In one embodiment, the first UV blocker may include at least one of methylene bis-benzotriazolyl tetramethylbutylphenol, tris-biphenyl triazine, phenylbenzimidazole sulfonic acid, terephthalylidene dicamphor sulfonic acid, titanium dioxide, and bis-ethylhexyloxyphenol methoxyphenyl triazine. Specifically, it may include at least one of methylene bis-benzotriazolyl tetramethylbutylphenol and tris-biphenyl triazine.

In one embodiment, the first UV blocker may be contained in an amount of 0.01 to 15 wt%, specifically 0.1 to 10 wt%, more specifically 0.5 to 5 wt%, based on the total weight of the cosmetic composition.

In one embodiment, the second UV blocker may be contained in the oil phase part. As the second UV blocker is stably contained in the oil phase part, it may exhibit an excellent effect of blocking harmful radiation.

In one embodiment, the second UV blocker may include at least one of ethylhexyl triazone, diethylaminohydroxybenzoyl hexyl benzoate, bis-ethylhexyloxyphenol methoxyphenyl triazine, ethylhexyl methoxycinnamate, ethylhexyl salicylate, octocrylene, diethylhexyl butamido triazone, homosalate, isoamyl p-methoxycinnamate, and polysilicone-15. Specifically, it may include at least one of ethylhexyl triazone, diethylaminohydroxybenzoyl hexyl benzoate, polysilicone-15, and bis-ethylhexyloxyphenol methoxyphenyl triazine.

In one embodiment, the second UV blocker may be contained in an amount of 0.1 to 30 wt%, specifically 0.5 to 20 wt%, more specifically 1 to 15 wt%, based on the total weight of the cosmetic composition.

In one embodiment, the third UV blocker may be contained in the oil phase part. As the third UV blocker is stably dispersed in the oil phase part, it may exhibit an excellent effect of blocking harmful radiation.

In one embodiment, the third UV blocker may include an inorganic UV blocker. In a specific embodiment, the inorganic UV blocker may include at least one of titanium dioxide and zinc oxide. In a specific embodiment, the third UV blocker may be provided after being physically or chemically modified with a modifier. Examples of the modifier include, but are not limited to, aluminum hydroxide, triethoxycaprylylsilane, and stearic acid.

In one embodiment, the third UV blocker may be contained in an amount of 0.1 to 50 wt%, specifically 0.5 to 45 wt%, more specifically 1 to 40 wt%, based on the total weight of the cosmetic composition.

In one embodiment, the emulsifier may serve to form stable emulsion particles, improve the dispersibility of the third UV blocker, and improve the stability of the first UV blocker and the second UV blocker in the emulsion formulation, thereby maintaining an excellent blocking effect against harmful radiation for a long period of time.

In one embodiment, the emulsifier may include a polyglyceryl-based emulsifier. The polyglyceryl-based emulsifier may make the size of emulsion particles small and uniform, and may interact with the thickener contained in the aqueous phase part, thereby preventing phase separation resulting from aggregation of emulsion particles even during long-term storage, thus forming a stable emulsion.

In one embodiment, the polyglyceryl-based emulsifier may include at least one of polyglyceryl-5 polyricinoleate, polyglyceryl-6 polyricinoleate, polyglyceryl-2 dipolyhydroxystearate, polyglyceryl-4 isostearate, polyglyceryl-4 diisostearate/polyhydroxystearate/sebacate, polyglyceryl-2 oleate, polyglyceryl-4 oleate, polyglyceryl-6 oleate, polyglyceryl-2 stearate, and polyglyceryl-4 stearate. Specifically, it may include polyglyceryl-5 polyricinoleate.

In one embodiment, the emulsifier may be contained in an amount of 0.1 to 7.0 wt%, specifically 0.5 to 6.0 wt%, more specifically 1.5 to 5.0 wt%, based on the total weight of the cosmetic composition. If the emulsifier is contained in an amount of less than 0.1 wt%, emulsion particles may not be formed smoothly, and if the emulsifier is contained in an amount of more than 7.0 wt%, the viscosity of the cosmetic composition may be significantly reduced, making it unsuitable for application.

In one embodiment, the thickener may serve to further enhance the stability of emulsion particles.

In a specific embodiment, as the thickener is contained in the aqueous phase part of the cosmetic composition in emulsion form, it may stabilize the first UV blocker component, etc. contained in the aqueous phase part and contribute to the formation and stabilization of emulsion particles.

In one embodiment, the thickener may include at least one inorganic thickener selected from among saponite, montmorillonite, smectite, kaolin, illite, hectorite, bentonite, and magnesium aluminum silicate. Specifically, it may include hectorite.

In one embodiment, the thickener may be contained in an amount of 0.01 to 3.0 wt%, specifically 0.05 to 2.0 wt%, more specifically 0.1 to 1.0 wt%, based on the total weight of the cosmetic composition. When the thickener is contained in the above amount, the stability of the formulation may be further improved.

In one embodiment, the cosmetic composition of the present invention may contain the emulsifier and the thickener at a weight ratio of 0.5:1 to 10:1. When the emulsifier and the thickener are contained at the above weight ratio, stable emulsion particles may be formed, and a cosmetic composition having a viscosity suitable for application may be formed.

In one embodiment, the cosmetic composition in emulsion form having an excellent blocking effect against harmful radiation including UV radiation according to the present invention may further contain an oil, a preservative, or functional ingredients.

The oil acts as a solvent for the oil phase part, maintains the moisture of the skin, and may provide a continuous moisturizing feeling. As the oil, silicone-based oil, hydrocarbon-based oil, ester-based oil, or the like may be used. However, the oil is not limited thereto, and any oil that may be generally used in the art may be appropriately selected without limitation. Specific examples of the oil include ester-based oils such as C12-15 alkyl benzoate, dibutyl adipate, butylene glycol dicaprylate/dicaprate, ethylhexyl stearate, and propylheptyl caprylate; silicone-based oils such as disiloxane, caprylyl methicone, and methyl trimethicone; and hydrocarbon-based oils such as isododecane, hydrogenated poly(C6-14 olefin), and C15-19 alkane.

The preservative is used to enhance the preservation effect, and for example, at least one of ethylhexylglycerin, 1,2-hexanediol, caprylyl glycol, phenoxyethanol, and glyceryl caprylate may be used, but the preservative is not limited thereto, and any preservative that may be generally used in the art may be appropriately selected without limitation.

As the functional ingredients, a mixture of various ingredients may be added depending on the purpose of the cosmetic composition. The functional ingredients include, for example, an anti-wrinkle agent, a whitening functional ingredient, an anti-pigmentation ingredient, a skin soothing functional ingredient, and a sebum or pore care functional ingredient.

In addition, the cosmetic composition in emulsion form having an excellent blocking effect against harmful radiation including UV radiation according to the present invention may further contain ingredients that are contained in general cosmetic compositions, and these ingredients include, for example, polyols, functional ingredients, a feeling-regulating agent, an emulsifying stabilizer, a skin conditioning agent, a chelating agent, a sterilizing agent, a pH regulator, an antioxidant, an alcohol, plant extracts, fragrances, an emollient, etc.

The contents of the above-described ingredients are not particularly limited, and may be easily selected by those skilled in the art within a range that does not impair the purpose and effect of the present invention.

In one embodiment, the cosmetic composition in emulsion form having an excellent blocking effect against harmful radiation including UV radiation according to the present invention may be of a water-in-oil type. The term "water-in-oil type" refers to a state in which an aqueous phase is dispersed in the form of particles in an oil phase, and may include not only a water-in-oil (W/O) emulsion but also a multiple emulsion such as oil-in-water-in-oil (O/W/O). When the cosmetic composition in emulsion form is prepared as a water-in-oil type emulsion in which the outer continuous phase is an oil phase, it may have better long-lasting water resistance.

In addition, the cosmetic composition in emulsion form having an excellent blocking effect against harmful radiation including UV radiation according to the present invention may be provided in a form that is commonly prepared in the art. For example, it may be formulated in the form of an oil-in-water (O/W) or water-in-oil-in-water (W/O/W) type emulsion. However, the present invention is not limited thereto, and the composition may also be provided in various forms such as solutions, suspensions, emulsions, solids, oils, powders, pastes, aerosol foams, mists, etc.

In one embodiment, the cosmetic composition may be provided in the form of a gel, lotion, cream, low-viscosity liquid, or fluid including a water-in-oil emulsion. Specifically, it may be provided in the form of a lotion.

In one embodiment, the cosmetic composition of the present invention may be manufactured into various cosmetics having an excellent effect of blocking harmful radiation including UV radiation. The cosmetics include, for example, sun care products such as sun cream, sun stick, sun milk, sun cushion, sun spray, and sun lotion, and may be manufactured as functional cosmetics such as nourishing cream, essence, ampoule, moisturizing cream, and eye cream, or basic cosmetics such as skin toner and lotion. However, the present invention is not limited thereto.

Hereinafter, examples and experimental examples will be presented to explain the present invention in more detail, but the present invention is not limited thereto.

### Experimental Example 1. Experiment for Comparison According to Weight Ratio between Emulsifier and Thickener

### (1) Preparation of Examples and Comparative Examples

In the present invention, in order to confirm the optimal weight ratio between an emulsifier and a thickener, water-in-oil type emulsions of Examples and Comparative Examples were prepared as shown in Table 1 below. Specifically, polyglyceryl-5 polyricinoleate was used as an emulsifier, hectorite was used as an aqueous phase thickener, methylene bis-benzotriazolyl tetramethylbutylphenol was used as a first UV blocker, ethylhexyl triazone, diethylaminohydroxybenzoyl hexyl benzoate, polysilicone-15 and bis-ethylhexyloxyphenol methoxyphenyl triazine were used as a second UV blocker, and titanium dioxide and zinc oxide were used as a third UV blocker. The unit in Table 1 below is wt%.

**[Table 1]**

| **Phase** | **Component** | **Example** | | | **Comparative Example** | |
|---|---|---|---|---|---|---|
| | | **1** | **2** | **3** | **1** | **2** |
| **A (oil phase)** | Ethylhexyl triazone | 3.00 | | | | |
| | Diethylaminohydroxybenzoyl hexyl benzoate | 3.50 | | | | |
| | Bis-ethylhexyloxyphenol methoxyphenyl triazine | 2.00 | | | | |
| | Polysilicone-15 | 3.00 | | | | |
| | C12-15 alkyl benzoate | 5.00 | | | | |
| | Dibutyl adipate | 10.00 | | | | |
| | Glyceryl caprylate | 0.100 | | | | |
| | Ethylhexylglycerin | 0.300 | | | | |
| **B (oil phase)** | Disiloxane | 4.00 | | | | |
| | Propylheptyl caprylate | 12.00 | | | | |
| | Isododecane | 4.00 | | | | |
| | Disteardimonium hectorite | 1.50 | | | | |
| **C (oil phase)** | Polyglyceryl-5 polyricinoleate | **5.00** | **3.00** | **1.50** | **8.00** | - |
| | Tocopherol | 0.50 | | | | |
| **D (oil phase)** | Titanium dioxide | 12.00 | | | | |
| | Zinc oxide | 10.00 | | | | |
| **E (aqueous phase)** | Hectorite | **0.50** | **0.60** | **1.50** | **0.70** | **1.00** |
| | Methylene bis-benzotriazolyl tetramethylbutylphenol | 3.00 | | | | |
| | 1,2-hexanediol | 0.50 | | | | |
| | Propanediol | 10.00 | | | | |
| | Water | To 100 | | | | |
| **Emulsifier: thickener (weight ratio)** | | **10:1** | **5:1** | **1:1** | **11:1** | **0:1** |

The specific preparation method is as follows.

First, phase A was dissolved in an oil-phase beaker at about 80°C, phases B to D were additionally mixed and dispersed therein, and phase E was dispersed in a water-phase beaker at room temperature. The dispersion in the oil-phase beaker in which phases A to D have been dispersed was stirred using a homomixer at 4,000 rpm for 6 minutes, and then phase E was slowly added to the oil-phase beaker. Thereafter, the mixture was stirred using a homomixer at 5,600 rpm for an additional 8 minutes, thereby completing the Examples and the Comparative Examples.

### (2) Formulation Stability Evaluation and Viscosity Measurement

For the Examples and Comparative examples prepared using the components and contents shown in Table 1 above, formulation stability evaluation and viscosity measurement were performed. The specific experimental method is as follows.

For formulation stability evaluation, it was observed whether a uniform water-in-oil type formulation was formed without separation between the oil phase and the aqueous phase with the naked eye, and whether agglomeration occurred. In addition, the formulation was stored for 3 months under high temperature (40°C), room temperature (25°C), and low temperature (5°C) conditions and the condition under which the process of reducing the temperature from high temperature, room temperature and low temperature and increasing from low temperature to room temperature and high temperature was repeated, and it was observed whether the formulation immediately after preparation was still maintained. If the formulation stability was not maintained in any of the above 4 temperature conditions, it was judged as failed.

For viscosity measurement, the viscosity of each of the Examples and the Comparative Examples was measured using a Brookfield viscometer LVT under the conditions of LV 4 pin, 6 rpm, and 1 min at room temperature (25°C), and the measured viscosities are shown in Table 2 below.

### (3) Measurement of Harmful Radiation Blocking Effect and Measurement of Water Resistance

To evaluate the harmful radiation blocking effect and water resistance of each of the Examples and the Comparative Examples, SPF measurements were conducted *in vitro.*

Specifically, for the measurement of the harmful radiation blocking effect, 1.3 mg/cm² of each of the Examples and the Comparative Examples were taken and evenly applied to a PMMA plate for 30 seconds, and then dried in a dark room for 15 minutes, thereby preparing samples. The *in vitro* SPF value before immersion in water was measured using a Labsphere UV 2000S *in vitro* SPF analyzer. The sun protection factor was expressed as the average value of three measurements, and the measurement results are shown in Table 2 below. In addition, FIG. 1a shows the absorbance of Example 1 for harmful radiation with a wavelength ranging from 250 to 450 nm, and FIG. 1b shows the absorbance of Comparative Example 1 for harmful radiation with a wavelength ranging from 250 to 450 nm.

In addition, for the measurement of water resistance, 1.3 mg/cm² of each of the Examples and the Comparative Examples were taken and evenly applied to a PMMA plate for 30 seconds as in the measurement of the harmful radiation blocking effect. Then, the plate was attached to the bottom of a 1-L beaker, 600 ml of distilled water at 30°C was added thereto, and then stirred using an Agi mixer at a rotation speed of 180 rpm for 30 minutes, and then dried for 30 minutes, and the SPF value was measured. The SPF value was measured in the same manner as the method of measuring the harmful radiation blocking effect. The measurement results obtained using the measurement method are shown in Table 2 below.

**[Table 2]**

| | **Example** | | | **Comparative Example** | |
|---|---|---|---|---|---|
| | **1** | **2** | **3** | **1** | **2** |
| **Formulation stability** | O | O | O | O | X |
| **Viscosity (cP)** | 72,000 | 65,000 | 60,000 | 11,000 | Not measurable |
| **In vitro SPF** | 82 | 93 | 98 | 40 | Not measurable |
| **In vitro SPF (water resistance)** | 76 | 82 | 90 | 22 | Not measurable |

Referring to Table 2 above, it was confirmed that, in the case of Examples 1 to 3 containing the emulsifier and the thickener at an appropriate weight ratio, stable formulations having a viscosity suitable for application were formed, and they had a very high SPF value and also had excellent water resistance. In addition, referring to FIGS. 1a and 1b, it was confirmed that the absorbance of Example 1 for harmful radiation with a wavelength ranging from 250 to 450 nm was significantly superior to that of Comparative Example 1. On the other hand, it was confirmed that, in the case of Comparative Example 1 containing the emulsifier in an excessive amount compared to the weight of the thickener, a stable formulation was formed, but the viscosity significantly decreased, making it unsuitable for application, and the SPF value was also significantly low. It was confirmed that, in the case of Comparative Example 2 containing no emulsifier, the formulation itself was not formed, and thus viscosity and harmful radiation blocking effect were not measurable.

Therefore, it was confirmed that containing the emulsifier and the thickener at an appropriate weight ratio is important in the preparation of the cosmetic composition in emulsion form having an excellent effect of blocking harmful radiation including UV radiation according to the present invention.

### Experimental Example 2. Experiment for Comparison According to Type of Thickener and Whether Thickener Is Contained in an Aqueous phase Part

### (1) Preparation of Comparative Examples

In the present invention, in order to evaluate the effect according to the type of thickener and the phase in which the thickener is contained, Comparative Examples were prepared as shown in Table 3 below according to the same preparation method as in Experimental Example 1. Specifically, the thickener (hectorite) of phase E as in Example 1 was contained in phase B, or the component of the thickener of phase E was changed to disteardimonium hectorite, and the remaining components and contents were the same as those in Example 1. The contents in Table 3 below are in wt%.

**[Table 3]**

| **Phase** | **Component** | **Comparative Example** | |
|---|---|---|---|
| | | **3** | **4** |
| **B (oil phase)** | Disteardimonium hectorite | - | - |
| | Hectorite | 1.00 | - |
| **E (aqueous phase)** | Hectorite | - | - |
| | Disteardimonium hectorite | - | 1.50 |

### (2) Formulation Stability Evaluation

It was confirmed that, in the case of Comparative Example 3, in which the thickener of the present invention was contained in the oil phase, the thickener was not properly dispersed in the oil phase and a homogeneous emulsion was not formed, so that a cosmetic composition in emulsion form was not formed. It was confirmed that, in the case of Comparative Example 4, in which the thickener was changed to a different component, the thickener was not stabilized in the aqueous phase and a homogeneous emulsion was not formed, so that, like Comparative Example 3, a cosmetic composition in emulsion form was not properly formed.

Therefore, it was confirmed that containing a specific thickener of the present invention in the aqueous phase is important in the preparation of the cosmetic composition in emulsion form having an excellent effect of blocking harmful radiation including UV radiation according to the present invention.

### Experimental Example 3. Experiment for Comparison According to Type of Emulsifier

### (1) Preparation of Examples and Comparative Examples

In the present invention, in order to evaluate the effect according to the type of emulsifier, Examples and Comparative examples were prepared as shown in Table 4 below according to the same preparation method as in Experimental Example 1. Specifically, only the emulsifier of phase C in Example 1 was changed to a different component, and the remaining components and contents were the same as those in Example 1. The contents in Table 4 below are in wt%.

**[Table 4]**

| **Phase** | **Component** | **Example** | | **Comparative Example** | |
|---|---|---|---|---|---|
| | | **4** | **5** | **5** | **6** |
| **C (oil phase)** | Polyglyceryl-6 polyricinoleate | 5.00 | - | - | - |
| | Polyglyceryl-2 stearate | - | 5.00 | - | - |
| | PEG-9 polydimethylsiloxyethyl dimethicone | - | - | 5.00 | - |
| | PEG-10 dimethicone | - | - | - | 5.00 |

### (2) Formulation Stability Evaluation and Viscosity Measurement

For the Examples and Comparative Examples prepared according to Table 4 above, formulation stability evaluation and viscosity measurement were performed in the same manner as in Experimental Example 1, and the results are shown in Table 5 below.

### (3) Measurement of Harmful Radiation Blocking Effect and Measurement of Water Resistance

For the Examples and Comparative Examples prepared according to Table 4 above, the harmful radiation blocking effect and the water resistance were measured, and the results are shown in Table 5 below.

**[Table 5]**

| | **Example** | | **Comparative Example** | |
|---|---|---|---|---|
| | **4** | **5** | **5** | **6** |
| **Formulation stability** | O | O | X | X |
| **Viscosity (cP)** | 52,000 | 89,000 | 95,000 | 87,000 |
| **In vitro SPF** | 76 | 68 | 38 | 32 |
| **In vitro SPF (water resistance)** | 65 | 60 | 20 | 18 |

Referring to Table 5 above, it was confirmed that, in the case of both Examples 4 and 5, which used the polyglyceryl-based emulsifier, like Example 1, stable compositions in emulsion form were formed, and they had an excellent effect of blocking harmful radiation and excellent water resistance. On the other hand, it was confirmed that, in the case of Comparative Examples 5 and 6, which used emulsifiers other than the polyglyceryl-based emulsifier, compositions in emulsion form were formed, but the stability thereof was very poor, and a phenomenon of phase separation was found within 3 days under all temperature conditions, and accordingly, the harmful radiation blocking effect was significantly poorer than that of the Examples. In addition, it was confirmed that, in the case of Comparative Example 5, the viscosity was somewhat higher than that of the Examples, resulting in poor spreadability.

Therefore, it was confirmed that containing a specific emulsifier of the present invention is important in the preparation of the cosmetic composition in emulsion form having an excellent effect of blocking harmful radiation including UV radiation according to the present invention.

### Experimental Example 4. Experiment for Comparison According to Whether At Least Three UV Blockers Are Contained

### (1) Preparation of Examples and Comparative Examples

In the present invention, in order to evaluate the effect according to whether at least three UV blockers are contained, Examples and Comparative Examples were prepared as shown in Tables 6 and 7 below according to the same preparation method as in Experimental Example 1. Specifically, based on Example 1 above, Example 6 in Table 7 is one in which the first UV blocker was changed to tris-biphenyl triazine, Example 7 is one in which the first UV blocker was changed to terephthalylidene dicamphor sulfonic acid and bis-ethylhexyloxyphenol methoxyphenyl triazine, Example 8 is one in which titanium dioxide was further added as the first UV blocker, Example 9 is one in which ethylhexyl triazone among the second UV blockers was changed to homosalate and ethylhexyl salicylate, and Example 10 is one in which bis-ethylhexyloxyphenol methoxyphenyl triazine among the second UV blockers was changed to homosalate. In addition, based on Example 1, Comparative Example 7 in Table 8 below is one in which the first UV blocker component was excluded, Comparative Example 8 is one in which the third UV blocker was excluded, and Comparative Example 9 is one in which the second UV blocker was excluded. The remaining components and contents were the same as those in Example 1. The contents in Tables 6 and 7 below are in wt%.

**[Table 6]**

| **Phase** | **Component** | **Example** | | | | |
|---|---|---|---|---|---|---|
| | | **6** | **7** | **8** | **9** | **10** |
| **A (oil phase)** | Ethylhexyl triazone | 3.00 | 3.00 | 3.00 | - | 4.00 |
| | Diethylaminohydroxybenzoyl hexyl benzoate | 3.50 | 3.50 | 3.50 | 3.50 | 1.00 |
| | Bis-ethylhexyloxyphenol methoxyphenyl triazine | 2.00 | 2.00 | 2.00 | 1.00 | - |
| | Polysilicone-15 | 3.00 | 3.00 | 3.00 | - | - |
| | Homosalate | - | - | - | 3.00 | 3.00 |
| | Ethylhexyl salicylate | - | - | - | 4.00 | - |
| **D (oil phase)** | Titanium dioxide | 12.00 | 12.00 | 12.00 | 12.00 | 9.00 |
| | Zinc oxide | 10.00 | 10.00 | 10.00 | 10.00 | 25.00 |
| **E (aqueous phase)** | Methylene bis-benzotriazolyl tetramethylbutylphenol | - | - | 1.00 | 3.00 | 3.00 |
| | Tris-biphenyl triazine | 3.00 | - | - | - | - |
| | Terephthalylidene dicamphor sulfonic acid | - | 2.00 | - | - | - |
| | Bis-ethylhexyloxyphenol methoxyphenyl triazine | - | 0.50 | - | - | - |
| | Titanium dioxide | - | - | 1.50 | - | - |

**[Table 7]**

| **Phase** | **Component** | **Comparative Example** | | |
|---|---|---|---|---|
| | | **7** | **8** | **9** |
| **A (oil phase)** | Ethylhexyl triazone | 3.00 | 3.00 | - |
| | Diethylaminohydroxybenzoyl hexyl benzoate | 3.50 | 3.50 | - |
| | Bis-ethylhexyloxyphenol methoxyphenyl triazine | 2.00 | 2.00 | - |
| **D (oil phase)** | Titanium dioxide | 12.00 | - | 12.00 |
| | Zinc oxide | 10.00 | - | 10.00 |
| **E (aqueous phase)** | Methylene bis-benzotriazolyl tetramethylbutylphenol | - | 3.00 | 3.00 |

### (2) Formulation Stability Evaluation and Viscosity Measurement

For the Examples and Comparative Examples prepared according to Tables 6 and 7 above, formulation stability evaluation and viscosity measurement were performed in the same manner as in Experimental Example 1, and the results are shown in Table 8 below.

### (3) Measurement of Harmful Radiation Blocking Effect and Measurement of Water Resistance

For the Examples and Comparative Examples prepared according to Tables 6 and 7 above, the harmful radiation blocking effect and the water resistance were measured, and the results are shown in Table 8 below.

**[Table 8]**

| | **Example** | | | | | **Comparative Example** | | |
|---|---|---|---|---|---|---|---|---|
| | **6** | **7** | **8** | **9** | **10** | **7** | **8** | **9** |
| **Formulation stability** | O | O | O | O | O | O | X | O |
| **Viscosity (cP)** | 64,00 0 | 73,00 0 | 66,00 0 | 52,00 0 | 76,00 0 | 40,00 0 | 30,00 0 | 97,000 |
| **In vitro SPF** | 97 | 82 | 80 | 77 | 95 | 35 | 25 | 30 |
| **In vitro SPF (water resistance)** | 90 | 76 | 70 | 65 | 90 | 33 | 22 | 28 |

Referring to Table 8 above, it was confirmed that, in the case of Examples 6 to 10, in which three UV blockers of the present invention were all contained, stable compositions in emulsion form were formed and they showed excellent SPF values and water resistance, indicating that they had an excellent effect of blocking harmful radiation, whereas the Comparative Examples, in which any one of the three UV blockers was not contained, had a significantly reduced SPF value compared to the Examples. In addition, it was confirmed that Comparative Example 7 or 8, which did not contain the first UV blocker or the third UV blocker, had a viscosity that was somewhat lower than that of the Examples, and Comparative Example 9, which did not contain the second UV blocker, had a viscosity that was somewhat higher than that of the Examples, and thus was unsuitable for application. It was confirmed that, in the case of Comparative Example 8, which did not contain the third UV blocker, phase separation occurred when stored at high temperature, indicating that the formulation stability was also poor.

Therefore, it was confirmed that containing at least three UV blockers of the present invention is important in the preparation of the cosmetic composition in emulsion form having an excellent effect of blocking harmful radiation including UV radiation according to the present invention.

Although the present invention has been described in detail with reference to the specific features, it will be apparent to those skilled in the art that this detailed description is only of a preferred embodiment thereof, and does not limit the scope of the present invention. Thus, the substantial scope of the present invention will be defined by the appended claims and equivalents thereto.

## Claims

1. A cosmetic composition in emulsion form comprising an aqueous phase part and an oil phase part,
the cosmetic composition containing at least three UV blockers, an emulsifier, and a thickener,
wherein the at least three UV blockers include a first UV blocker, a second UV blocker, and a third UV blocker, thereby providing an excellent effect of blocking harmful radiation including UV radiation.

2. The cosmetic composition of claim 1, wherein the first UV blocker is contained in the aqueous phase part.

3. The cosmetic composition of claim 1, wherein the second UV blocker and the third UV blocker are contained in the oil phase part.

4. The cosmetic composition of claim 1, wherein the emulsifier and the thickener are present in a weight ratio from 0.5:1 to 10:1.

5. The cosmetic composition of claim 1, wherein the emulsifier comprises a polyglyceryl-based emulsifier.

6. The cosmetic composition of claim 5, wherein the polyglyceryl-based emulsifier comprises at least one of polyglyceryl-5 polyricinoleate, polyglyceryl-6 polyricinoleate, polyglyceryl-2 dipolyhydroxystearate, polyglyceryl-4 isostearate, polyglyceryl-4 diisostearate/polyhydroxystearate/sebacate, polyglyceryl-2 oleate, polyglyceryl-4 oleate, polyglyceryl-6 oleate, polyglyceryl-2 stearate, and polyglyceryl-4 stearate.

7. The cosmetic composition of claim 1, wherein the emulsifier is contained in an amount of 0.1 to 7.0 wt% based on the total weight of the cosmetic composition.

8. The cosmetic composition of claim 1, wherein the thickener is contained in the aqueous phase part.

9. The cosmetic composition of claim 1, wherein the thickener comprises at least one inorganic thickener selected from among saponite, montmorillonite, smectite, kaolin, illite, hectorite, bentonite, and magnesium aluminum silicate.

10. The cosmetic composition of claim 1, wherein the thickener is contained in an amount of 0.01 to 3.0 wt% based on the total weight of the cosmetic composition.

11. The cosmetic composition of claim 1, wherein the first UV blocker comprises at least one of methylene bis-benzotriazolyl tetramethylbutylphenol, tris-biphenyl triazine, phenylbenzimidazole sulfonic acid, terephthalylidene dicamphor sulfonic acid, titanium dioxide, and bis-ethylhexyloxyphenol methoxyphenyl triazine.

12. The cosmetic composition of claim 1, wherein the second UV blocker comprises at least one of ethylhexyl triazone, diethylamino hydroxybenzoyl hexyl benzoate, bis-ethylhexyloxyphenol methoxyphenyl triazine, ethylhexyl methoxycinnamate, ethylhexyl salicylate, octocrylene, diethylhexyl butamido triazone, homosalate, isoamyl p-methoxycinnamate, and polysilicone-15.

13. The cosmetic composition of claim 1, wherein the third UV blocker comprises at least one of titanium dioxide and zinc oxide.

14. The cosmetic composition of claim 1, wherein the harmful radiation including UV radiation has a wavelength of 250 to 500 nm.
